# EUROPÄISCHE PATENTSCHRIFT

(11) **EP 2 756 288 B1**
(45) Veröffentlichungstag und Bekanntmachung des Hinweises auf die Patenterteilung: **07.06.2023**
(21) Anmeldenummer: 12761954.2
(22) Anmeldetag: 14.09.2012
(51) Int. Cl.: G01N 33/487, G01N 21/84

(54) **ANALYTISCHES TESTBAND FÜR EINE TESTBANDKASSETTE**
ANALYTICAL TEST TAPE FOR A TEST TAPE CASSETTE
BANDE DE TEST ANALYTIQUE POUR UNE CASSETTE DE BANDE

(30) Priorität: 16.09.2011 EP 11181718
(43) Veröffentlichungstag der Anmeldung: 23.07.2014
(73) Patentinhaber: Roche Diabetes Care GmbH, 68305 Mannheim (DE); F. Hoffmann-La Roche AG, 4070 Basel (CH)
(72) Erfinder: DREIBHOLZ, Joerg, 67122 Altrip (DE); FREITAG, Christian, 55278 Weinolsheim (DE); JAECK, Thomas, 68542 Heddesheim (DE); PACHL, Rudolf, 67158 Ellerstadt (DE); SCHMIDTCHEN, Elke, 68199 Mannheim (DE); KETH, Ingrid, 67551 Worms (DE); SCHWOEBEL, Wolfgang, 68309 Mannheim (DE); SEELIG, Peter, 60596 Frankfurt (Main) (DE)
(74) Vertreter: Pfiz, Thomas
(86) Internationale Anmeldenummer: PCT/EP2012/068145
(87) Internationale Veröffentlichungsnummer: WO 2013/037969

(56) Entgegenhaltungen:
- EP-A1- 1 424 040
- EP-A1- 2 011 630
- EP-A1- 2 116 180
- EP-A1- 2 151 686
- EP-A1- 2 223 746
- EP-A2- 1 702 565
- WO-A1-2005/032372

## Beschreibung

Die Erfindung betrifft ein analytisches Testband, insbesondere zum Einsatz in einer Testbandkassette, mit einem auf einer Spule aufwickelbaren Trägerband und einer Vielzahl von auf dem Trägerband in Bandlängsrichtung verteilt angeordneten Testelementen, welche ein Spreitgewebe zur Applikation von Körperflüssigkeit und eine darunter liegende Reagenzschicht zum Nachweis eines Analyten in der Körperflüssigkeit aufweisen, wobei das Spreitgewebe aus gitterförmig verkreuzten Gewebefäden gebildet ist, wobei die Biegesteifigkeit des Spreitgewebes bereichsweise modifiziert ist, so dass die Biegesteifigkeit in Bandquerrichtung hoch und in Bandlängsrichtung niedrig ist.

Beispiele für Testbänder in Testbandkassetten mit einem Spreitgewebe finden sich in EP1424040 und EP2223746.

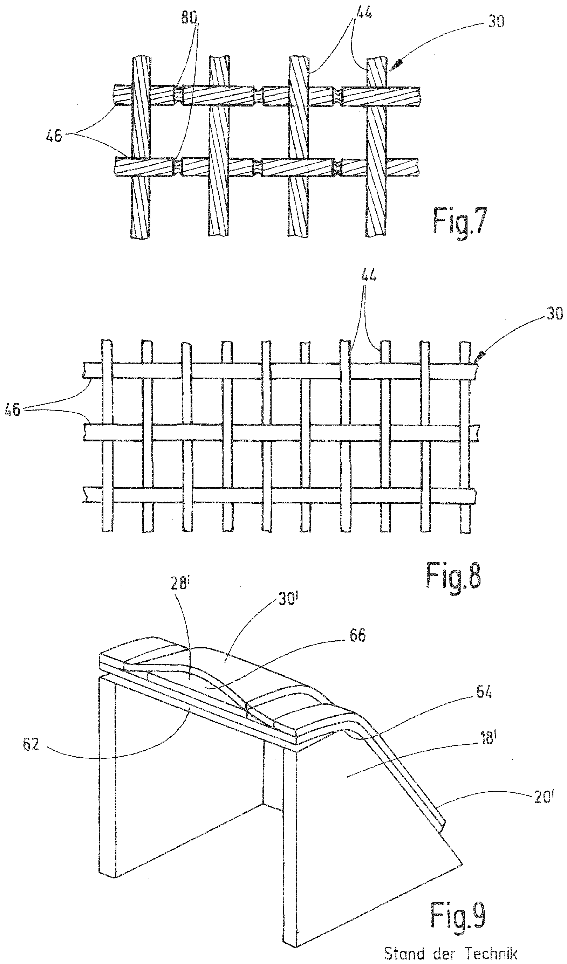

Bei einer aus der EP-A 1 878 379 bekannten Testbandkassette dieser Art weist die Bandführung an einem Messkopf einen ebenen Auflagerahmen auf, welcher ein Testelement an der Messstelle plan aufspannt. Das Testband wird dabei ausgehend von Umlenkschrägen über die in Bandquerrichtung verlaufenden Rahmenschenkel abgeknickt, um eine frei gespannte Planlage zu erreichen. Die zur Messung eingesetzten etikettenartigen Testelemente bzw. Testfelder weisen einen zentralen Chemieträger auf, der an seinen Seitenrändem von dem Spreitgewebe hintergriffen wird. Als möglicher Nachteil hat sich dabei herausgestellt, dass unter den herrschenden Belastungen des Testbandaufbaus auf dem ebenen Auflagerahmen eine Spaltbildung zwischen dem Spreitnetz und dem Chemieträger auftreten kann, wie es in der Zeichnung in Fig. 9 veranschaulicht ist. Das Spaltmaß ist dabei im Zentrum maximal und fällt zu den Seiten hin ab, was unterschiedliche Kapillarkräfte erzeugen kann und letztlich unerwünschte Verteilungen (ggf. mit Vorzugsrichtung) des Messmediums zur Folge haben kann.

Insbesondere wurde festgestellt, dass eine solche Spaltbildung die Neigung zu einer "Entnetzung", d.h. einer Migration der Blutprobe aus den benetzten Gewebemaschen und die Empfindlichkeit gegenüber Verunreinigungen (z.B. Glucose) auf der Haut des Probanden verstärkt. Der letztgenannte Effekt kann sich dadurch ergeben, dass das durch einen Hauteinstich gewonnene Kapillarblut einen Blutstropfen an der Einstichstelle bildet, der zunächst in seinem im Hautkontakt stehenden Randbereich mit Verschmutzungen aufkonzentriert wird. Das "native" Blut wird dann aufgrund der Spaltbildung im Spreitgewebe zunächst in die Randbereiche des Chemieträgers verteilt, während das nachströmende verschmutzte Blut dann auf den zentralen Messfleck gelangt, wodurch die Messperformance beeinträchtigt werden kann.

Ausgehend hiervon liegt der Erfindung die Aufgabe zugrunde, die im Stand der Technik bekannten Testbandsysteme und darin eingesetzten disposiblen Testmittel zu optimieren und eine weiter verbesserte Zuverlässigkeit und Genauigkeit der Messung sicherzustellen.

Zur Lösung dieser Aufgabe wird die im Patentanspruch 1 angegebene Merkmalskombination vorgeschlagen. Vorteilhafte Ausgestaltungen und Weiterbildungen der Erfindung ergeben sich aus den abhängigen Ansprüchen.

Gemäß einem bevorzugten Aspekt der Erfindung ist ein analytisches Testband, insbesondere zum Einsatz in einer Testbandkassette vorgesehen, bei dem die Biegesteifigkeit des Spreitgewebes durch uneinheitliche Eigenschaftsänderungen der Gewebefäden bereichsweise bzw. lokal modifiziert ist, so dass die Biegesteifigkeit in Bandquerrichtung hoch und in Bandlängsrichtung niedrig ist, wobei die Biegesteifigkeit durch einer der folgenden Möglichkeiten modifiziert ist:
- der Einsatz unterschiedlich starker Gewebefäden in entsprechenden Bereichen des Gewebes;
- das Herauslösen einzelner Gewebefäden in bestimmten Bereichen;
- die zusätzliche Beschichtung in bestimmten Bereichen der Gewebefäden;
- die Verdünnung oder lokale Schwächung von einem oder mehreren Gewebefäden;
- der Einsatz multifiler Gewebefäden.

Damit können die richtungsabhängigen Beanspruchungen im Bereich der Applikationsspitze zur Vermeidung einer Spaltbildung entsprechend ausgeglichen bzw. berücksichtigt werden. Dies lässt sich dahingehend optimieren, dass die Biegesteifigkeit des Spreitgewebes zur Reduzierung des Abstandes zwischen dem Spreitgewebe und der Reagenzschicht eines zur Applikation von Körperflüssigkeit bereitgestellten Testfeldes angepasst wird.

Eine vorteilhafte Möglichkeit für eine solche nicht-isotrope, bereichsweise Gewebemodifikation besteht darin, dass die Gewebefäden vorzugsweise durch Laserablation oder Ätzen oder mechanischen Materialabtrag stellenweise reduzierte Fadenquerschnitte aufweisen.

Alternativ oder ergänzend ist es auch möglich, dass die Gewebefäden durch unterschiedliche Fadenstärken, Beschichtungen oder Filamentstrukturen zur Modifikation der Biegesteifigkeit uneinheitlich ausgebildet sind.

Die Biegesteifigkeit lässt sich auch dadurch gezielt anpassen, dass die Gewebegeometrie des Spreitgewebes beispielsweise durch Herauslösen einzelner Gewebefäden lokal variiert wird.

Gemäß einer weiteren bevorzugten Ausgestaltung ist das Spreitgewebe breiter als die Reagenzschicht und im Bereich seiner überstehenden Seitenränder durch Abstandshalter eben auf dem Testband abgestützt. Dadurch lassen sich mit einer Gewebekrümmung einhergehende unerwünschte Spaltbildungen weiter unterdrücken.

Um einen guten Kompromiss zwischen einer für die Probenverteilung hinreichenden Mindestdicke und dem Radius des aufgewickelten und verbrauchten Testbandes zu finden, ist es vorteilhaft, wenn das Spreitgewebe eine Dicke von weniger als 150, vorzugsweise weniger als 110 µm aufweist.

Ein weiterer, nicht beanspruchter Aspekt der Erfindung geht von dem Gedanken aus, die Bandführungsgeometrie bzw. den Testfeldaufbau so zu gestalten, dass eine Spaltbildung zwischen Spreitgewebe und Chemieträger unter Einsatzbedingungen weitgehend minimiert wird. Dementsprechend wird gemäß diesem Aspekt der Erfindung vorgeschlagen, dass die Applikationsspitze eine längs des Testbandes (bzw. in Bandlaufrichtung) bogenförmig verlaufende und quer dazu ungekrümmte Führungsbahn zur knickfreien Abstützung des Testbands aufweist, und dass die Führungsbahn in ihrem Scheitelbereich einen zentralen Durchbruch als Messfenster für eine optische Messung an den Testelementen begrenzt. Durch die bogenförmige Längskrümmung der Führungsbahn bzw. Lauffläche kann der Biegesteifigkeit der Testelemente und insbesondere des Spreitgewebes durch einen gleichmäßig abstützenden mechanischen Unterbau Rechnung getragen werden, so dass scharfe Bandknicke und damit eine Spaltbildung vermieden werden. Um Verschiebungen in dem Mehrschichtaufbau zu vermeiden, erfolgt die Bandkrümmung nur eindimensional, während in Bandquerrichtung eine ungekrümmte Abstützung erreicht wird. Zugleich kann durch die Bogenform der Führungsbahn eine gezielte Probenaufnahme auch kleinster Probenmengen im Scheitelpunkt sichergestellt werden, wobei durch das dort positionierte, als lichte Öffnung ausgebildete Messfenster auch eine Reduzierung der erforderlichen Testfeldfläche möglich ist.

Eine weitere Verbesserung in dieser Hinsicht lässt sich dadurch erreichen, dass die kreisbogenförmige Führungsbahn einen festen Krümmungsradius vorzugsweise im Bereich von 3 bis 5 mm aufweist, und dass die Führungsbahn eine Längserstreckung in Bandtransportrichtung im Bereich von 5 bis 8 mm aufweist. Dabei ist es günstig, wenn die Längserstreckung der Führungsbahn gleich oder kleiner als die vorzugsweise im Bereich von 5 bis 15 mm liegende Länge der Testelemente ist.

Die Einsatzbedingungen und insbesondere die Bandzugkraft sollten so angepasst sein, dass das Testband unter Zugspannung flächig auf der Führungsbahn abgestützt ist, so dass das Spreitgewebe im Wesentlichen spaltfrei auf der Reagenzschicht anliegt.

Um eine Spaltbildung unter dem unverklebten Zentralbereich des Spreitgewebes zu vermeiden, ist es vorteilhaft, wenn die Reagenzschicht in Bandquerrichtung gesehen schmaler als das Testband und breiter als das Messfenster ist.

Für eine optimierte rückseitige optische Messwerterfassung ist es von Vorteil, wenn eine die Führungsbahn bildende Gehäusewand der Applikationsspitze rückseitig zu dem Messfenster hin abgeschrägt ist.

Um Gebrauchs- und Herstellungsvorteile zu erreichen, ist es vorteilhaft, wenn die Applikationsspitze vorzugsweise als Spritzgusspartie einstückig an dem Kassettengehäuse angeformt ist und im Gebrauchszustand zum punktuellen Applizieren von Körperflüssigkeit aus dem Handgerät herausragt.

Im Folgenden wird die Erfindung anhand der in der Zeichnung schematisch dargestellten Ausführungsbeispiele näher erläutert. Es zeigen
- Fig. 1: ein Handgerät für Blutzuckertests mit einer als Verbrauchsmittel eingesetzten Testbandkassette in einer teilweise aufgebrochenen Seitenansicht;
- Fig. 2: einen Ausschnitt eines Testbands der Testbandkassette mit einem analytischen Testelement in einer perspektivischen Darstellung;
- Fig. 3: ein Gehäuseteil der Testbandkassette in einer perspektivischen Darstellung;
- Fig. 4: eine Applikationsspitze der Testbandkassette in der Draufsicht, im Längsschnitt und im Querschnitt;
- Fig. 5 und 6: einen Schnitt entlang der Linie 5 - 5 und 6 - 6 in Fig. 4;
- Fig. 7: ein Spreitgewebe für ein analytisches Testelement mit Schwächungsstellen in einer ausschnittsweisen Draufsicht;
- Fig. 8: eine weitere Ausführungsform eines modifizierten Spreitgewebes;
- Fig. 9: ein Testfeld auf einer Applikationsspitze gemäß dem Stand der Technik in geschnittener perspektivischer Darstellung.

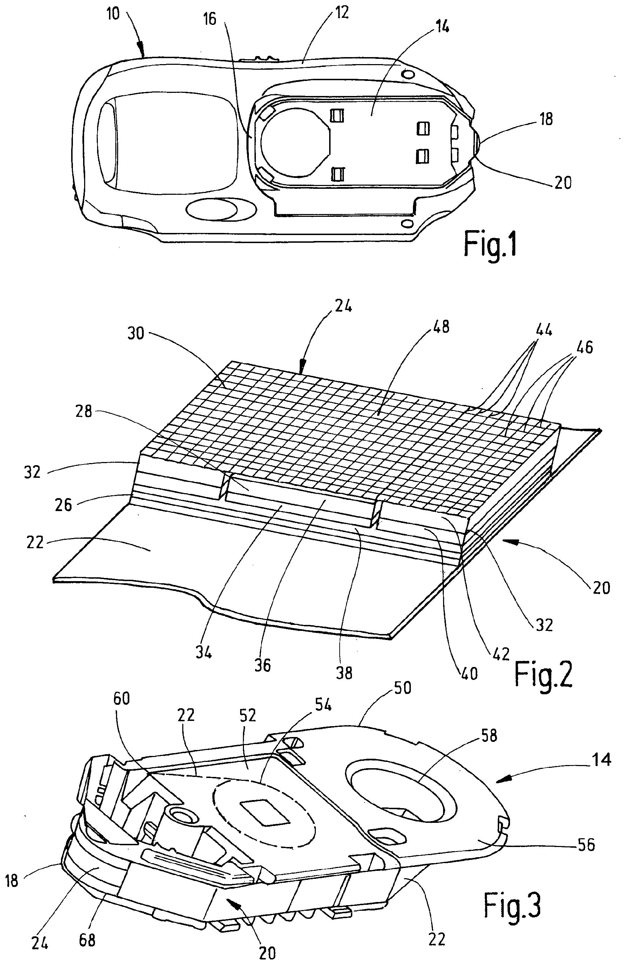

Das in Fig. 1 gezeigte Blutzuckermesssystem 10 ermöglicht speziell für insulinpflichtige Patienten die Durchführung von Glukosebestimmungen an vor Ort durch einen Hauteinstich gewonnenen Blutproben. Zu diesem Zweck ist das Handgerät 12 gleichsam als mobiles Labor in der Hand eines Probanden tragbar und benutzbar. Um die Handhabung weitgehend zu vereinfachen, lässt sich eine Testbandkassette 14 als analytisches Verbrauchsmittel zur Bevorratung einer Vielzahl von Einzeltests in einen Kassettenschacht 16 des Geräts 12 einsetzen, wobei im Gebrauchszustand (bei entfernter Schutzkappe) eine Applikationsspitze 18 frei aus dem Gerät 12 herausragt, so dass dort ein Testband 20 zum oberseitigen Auftrag eines Blutstropfens und zur rückseitigen photometrischen Untersuchung umgelenkt werden kann. Denkbar ist auch die Untersuchung anderer Körperflüssigkeiten, beispielsweise Gewebeflüssigkeit.

Fig. 2 zeigt einen Abschnitt des in der Testbandkassette 14 geführten Testbandes 20. Dieses umfasst ein aufwickelbares flexibles Transportband 22 und eine Vielzahl von darauf für einen sukzessiven Einmalgebrauch bevorrateten, in Bandlängsrichtung voneinander beabstandeten Testelementen 24. Beispielsweise besteht das Transportband 22 aus einer 5 mm breiten und 12 µm dicken Folie, auf die Testelemente 24 mit einer jeweiligen Gesamthöhe von etwa 200 µm aufgebracht sind.

Die mehrlagigen Testelemente 24 weisen als im Umriss rechteckförmige etikettenartige Flächengebilde einen auf das Transportband 22 aufgeklebten doppelseitigen Klebestreifen 26, einen darauf aufgebrachten Chemieträger 28 und ein den Chemieträger 28 an der von dem Transportband 22 abgewandten Oberseite überspannendes Spreitgewebe 30 für eine flächige Verteilung einer von oben auf das Spreitgewebe aufgetragenen Blutprobe auf. Seitlich neben dem Chemieträger 28 sind Abstandshalter 32 vorgesehen, um das Spreitgewebe 30 eben bzw. stufenfrei vollflächig abzustützen.

Der Chemieträger 28 umfasst eine lichtdurchlässige Trägerfolie 34 und eine darauf aufgebrachte Reagenzschicht 36, die aus einer oberen Pigmentschicht mit darunter liegendem Trockenchemiefilm in an sich bekannter Weise aufgebaut ist.

Die Abstandshalter 32 bestehen aus einem auf der oberen Klebeschicht 38 des doppelseitigen Klebestreifens 26 haftenden Basisstreifen 40 und einer darauf befindlichen Klebeschicht 42 zur seitlichen Fixierung des Spreitgewebes 30.

Das in Fig. 2 nur schematisch und in der Dicke nicht maßstäblich gezeigte Spreitgewebe 30 ist durch gitterförmig miteinander verkreuzte Gewebefäden 44, 46 gebildet. Diese können als Kettfäden 44 und Schussfäden 46 in Leinwandbindung miteinander verbunden sein und zur lokalen Modifikation der Biegesteifigkeit uneinheitlich ausgebildet sein, wie es weiter unten näher erläutert wird. Das Spreitgewebe 30, das eine Dicke von etwa 100 µm aufweist, sorgt aufgrund seiner kapillaren Zwischenräume für eine rasche Aufnahme der Flüssigprobe auf der freien Oberseite und für eine flächige Verteilung auf die darunter liegende Reagenzschicht 36.

Dabei wird durch den einseitigen Verschluss der Gewebeöffnungen mit dem Klebematerial der flankierenden Klebeschichten 42 eine Art von Wabenstruktur über den Abstandshaltern 32 gebildet, die einen Blutfluss zu den Seitenkanten des Testelements 24 verhindert. Somit erfolgt die Flüssigkeitsverteilung bzw. -spreitung gezielt in dem nicht verklebten Zentralbereich des Gewebes 30 über der Reagenzschicht 36, so dass auf die nach dem Stand der Technik beispielsweise als Wachsstreifen mittels Thermotransferdruck eigens aufgebrachten hydrophoben Randstreifen ohne Nachteil verzichtet werden kann.

Die Reagenzschicht 36 spricht mit ihrem Trockenchemiefilm insbesondere auf Enzymbasis auf einen Analyten (Glucose) durch Farbumschlag an, so dass eine reflexionsphotometrische Erfassung durch den transparenten Folienverbund 22, 26, 34 hindurch von der Rückseite des Testbands 20 her erfolgen kann.

Fig. 3 zeigt das Kassettengehäuse 50 der Testbandkassette 14 bei abgenommenem Gehäusedeckel. Das Gehäuse 52 umschließt eine Vorratskammer 50 zur abgedichteten Lagerung einer Vorratsspule 54 für unverbrauchtes Testband. An einem Gehäuseflansch 56 ist eine rotierend angetriebene Aufwickelspule 58 zum Aufwickeln von verbrauchtem Testband gelagert. Das Testband 20 wird somit über eine einheitlich durch das Kassettengehäuse 50 gebildete Bandführung von der Vorratsspule 54 abgezogen, über die Applikationsspitze 18 umgelenkt und auf der Aufwickelspule 58 entsorgt, wobei eine Durchzugdichtung 60 an der Vorratskammer 52 für die Aufrechterhaltung einer Bandspannung sorgt.

Die Testfelder 24 lassen sich somit durch Vorspulen des Transportbandes 22 an Applikationsspitze 18 nacheinander zum Einsatz bringen, um gezielt eine geringe Probenmenge auftragen zu können. Aufgrund der dabei ausgeübten Zugkraft ist der mehrlagige Bandaufbau richtungsabhängigen Dehnungen bzw. Kontraktionen insbesondere im Bereich von engen Umlenkstellen unterworfen.

Beim Stand der Technik, wie er in Fig. 9 für eine bekannte Applikationsspitze 18' mit ebenem Auflagerahmen 62 dargestellt ist, kann die Biegesteifigkeit des Spreitgewebes 30' in Bandlängs- und -querrichtung zu einem Abheben bzw. Aufwölben über dem Chemieträger 28' führen. Dieser Effekt beruht einerseits auf Biegungen an den engen Umlenkstellen 64 und auf der Bandwölbung in Querrichtung aufgrund der seitlichen Gewebeverklebung unterhalb der Höhe des Chemieträgers 28'. Dies führt zur Bildung eines Spalts 66 im Zentralbereich zwischen Spreitgewebe 30' und Chemieträger 28', wodurch der Blutübertrag und damit der der Messerfolg beeinträchtigt werden kann.

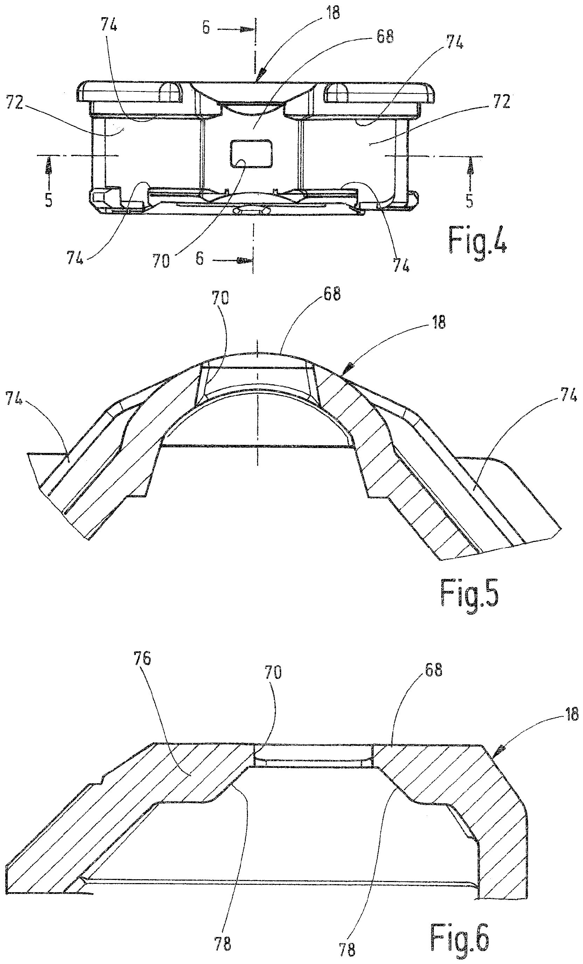

Um die vorstehend beschriebene Spaltbildung zu vermeiden, ist gemäß Fig. 4 bis 6 die Applikationsspitze 18 mit einer in Bandlängsrichtung bzw. Bandtransportrichtung bogenförmig verlaufenden, außen konvexen Führungsbahn 68 für das Testband 20 versehen. In ihrem Scheitelbereich begrenzt die Führungsbahn 68 einen zentralen Durchbruch 70 allseitig als Messfenster für eine rückseitige optische Vermessung der Testelemente 24.

Zur Ein- und Ausleitung des Testbands 20 an der Applikationsspitze 18 schließen sich an die Führungsbahn 68 endseitig Führungsschrägen 72 an, die einen spitzen Winkel einschließen. In diesem Bereich sind auch Seitenbegrenzungen 74 vorgesehen, die das Testband 20 gegen Querverschiebung sichern, während die Führungsbahn 68 frei von solchen Seitenbegrenzungen bleibt.

Wie am besten aus Fig. 5 ersichtlich, weist die kreisbogenförmige Führungsbahn 68 einen vorgegebenen, definierten Krümmungsradius auf, der zweckmäßig im Bereich von 3 bis 5 mm liegt. Entsprechend kann die Führungsbahn 68 eine Längserstreckung in Bandtransportrichtung im Bereich von 5 bis 8 mm besitzen. Dabei ist es zweckmäßig, wenn die Testelemente 24 an die Längserstreckung der Führungsbahn 68 angepasst sind. Besonders vorteilhaft sind die Testelemente 24 zur Vermeidung von Zug- und Schubspannungen durch Umlenkpunkte auf eine möglichst geringe Länge verkürzt.

Wie in Fig. 6 deutlich erkennbar, verläuft die Führungsbahn 68 in Bandquerrichtung gesehen ungekrümmt bzw. linear, so dass das Spreitgewebe 30 nur eindimensional in Bandlängsrichtung gebogen wird und dabei im Wesentlichen spaltfrei auf der Reagenzschicht 36 aufliegt. Zweckmäßig ist die Reagenzschicht 36 breiter als das Messfenster 70, so dass der Messfleck in jedem Fall auf der Reagenzschicht 36 liegt. Für den Strahlengang der photometrischen Messoptik ist die vorderseitig als Führungsbahn 68 ausgebildete Gehäusewand 76 der Applikationsspitze 68 an ihrer Rückseite 78 zu dem Messfenster 70 hin abgeschrägt.

Als weitere Maßnahme zur Vermeidung bzw. Reduzierung einer Spaltbildung zwischen dem Spreitgewebe 30 und der Reagenzschicht 36 ist die Biegesteifigkeit des Spreitgewebes 30 bereichsweise bzw. lokal entsprechend modifiziert.

Allgemein lässt sich die Biegesteifigkeit einer Gewebeprobe nach dem Freiträgerverfahren (Cantilever-Verfahren) bestimmen. Hierbei wird das Biegeverhalten aufgrund des Eigengewichts dadurch ermittelt, dass eine Biegelänge erfasst wird, bei der die Gewebeprobe unter ihrem Eigengewicht um einen definierten Winkel nach unten gebogen wird.

Beispielsweise kann eine hohe Biegesteifigkeit in Bandquerrichtung vorgesehen sein, um eine benötigte Planlage des Spreitgewebes sicherzustellen. Es kann aber auch eine niedrige Biegesteifigkeit in Bandlängsrichtung notwendig sein, um das Spreitgewebe umlenken zu können, ohne dass es zu ungewollten Aufwölbungen oder einer Delaminierung weiterer Teile des gesamten Testelementaufbaus kommt.

Ein isotropes Gewebe mit einheitlichen Schuss- und Kettfäden kann je nach Fadenstärke bzw. Gewebedicke nur eine in Bezug auf die gewünschte Testelementarchitektur gleichmäßig hohe oder niedrige Biegesteifigkeit besitzen.

Um erfindungsgemäß ein für die Testfunktion ausreichend dickes Spreitgewebe mit Bereichen hoher Biegesteifigkeit und zugleich mit Bereichen geringer Biegesteifigkeit zu erhalten, bestehen folgende Möglichkeiten, die Biegesteifigkeit bereichsweise bzw. lokal zu modifizieren:
- der Einsatz unterschiedlich starker Fäden in entsprechenden Bereichen des Gewebes;
- das Herauslösen einzelner Fäden in bestimmten Bereichen (nicht erfindungsgemäß);
- die zusätzliche Beschichtung in bestimmten Bereichen;
- die Verdünnung oder lokale Schwächung von einem oder mehreren Fäden;
- der Einsatz unterschiedlicher Fadenmaterialien (nicht erfindungsgemäß);
- der Einsatz unterschiedlicher Fadenbeschaffenheiten (z.B. multifile Fäden).

Fig. 7 zeigt ein Ausführungsbeispiel eines Spreitgewebes 30, bei dem nur eines der Fadensysteme 44, 46 (beispielsweise die Schussfäden 46) stellenweise reduzierte Fadenquerschnitte aufweist. Dies kann dadurch erzeugt werden, dass die Gewebefäden 46 in dem vorgefertigten Gewebe 30 durch Laserablation mit Schwächungsstellen 80 versehen werden. Denkbar sind auch Ätzverfahren oder ein mechanischer Materialabtrag beispielsweise mittels einer Wafersäge, um lokale Schwächungsstellen zur Reduzierung der Biegesteifigkeit einzubringen.

Fig. 8 veranschaulicht ein weiteres Ausführungsbeispiel, bei dem das Spreitgewebe 30 durch unterschiedliche Fadenstärken im Bereich seiner beiden Fadensysteme in der Biegesteifigkeit modifiziert ist. Dabei weisen die Kettfäden 44 eine geringere Fadenstärke und demzufolge eine geringere Biegesteifigkeit als die dickeren Schussfäden 46 auf. Möglich ist es auch, dass die Gewebegeometrie des Spreitgewebes 30 beispielsweise durch Herauslösen einzelner Gewebefäden lokal variiert wird.

## Patentansprüche

1. Analytisches Testband (20), insbesondere zum Einsatz in einer Testbandkassette (14), mit einem auf einer Spule aufwickelbaren Trägerband und einer Vielzahl von auf dem Trägerband in Bandlängsrichtung verteilt angeordneten Testelementen (24), welche ein Spreitgewebe (30) zur Applikation von Körperflüssigkeit und eine darunter liegende Reagenzschicht (36) zum Nachweis eines Analyten in der Körperflüssigkeit aufweisen, wobei das Spreitgewebe (30) aus gitterförmig verkreuzten Gewebefäden (44,46) gebildet ist wobei die Biegesteifigkeit des Spreitgewebes (30) bereichsweise modifiziert ist, so dass die Biegesteifigkeit in Bandquerrichtung hoch und in Bandlängsrichtung niedrig ist; wobei die Biegesteifigkeit durch eine der folgenden Möglichkeiten modifiziert ist:
- der Einsatz unterschiedlich starker Gewebefäden (44,46) in entsprechenden Bereichen des Gewebes;
- die zusätzliche Beschichtung in bestimmten Bereichen der Gewebefäden (44,46);
- die Verdünnung oder lokale Schwächung von einem oder mehreren Gewebefäden (44,46);
- der Einsatz multifiler Gewebefäden (44,46).

2. Analytisches Testband (20) nach Anspruch 1 **dadurch gekennzeichnet, dass** die Biegesteifigkeit des Spreitgewebes (30) zur Reduzierung des Abstandes zwischen dem Spreitgewebe (30) und der Reagenzschicht (36) eines zur Applikation von Körperflüssigkeit bereitgestellten Testfelds (20) angepasst ist.

3. Analytisches Testband (20) nach Anspruch 1 oder 2, **dadurch gekennzeichnet, dass** die Gewebefäden (44,46) vorzugsweise durch Laserablation oder Ätzen oder mechanischen Materialabtrag stellenweise reduzierte Fadenquerschnitte aufweisen.

4. Analytisches Testband (20) nach einem der Ansprüche 1 bis 3, **dadurch gekennzeichnet, dass** die Gewebefäden (44,46) durch unterschiedliche Fadenstärken, Beschichtungen oder Filamentstrukturen zur Modifikation der Biegesteifigkeit uneinheitlich ausgebildet sind.

5. Analytisches Testband (20) nach einem der Ansprüche 1 bis 4, **dadurch gekennzeichnet, dass** die Gewebegeometrie des Spreitgewebes (30) lokal variiert ist.

6. Analytisches Testband (20) nach einem der Ansprüche 1 bis 5, **dadurch gekennzeichnet, dass** das Spreitgewebe (30) breiter als die Reagenzschicht (36) ist und im Bereich seiner überstehenden Seitenränder durch Abstandshalter (32) eben auf dem Testband (20) abgestützt ist.

7. Analytisches Testband (20) nach einem der Ansprüche 1 bis 6, **dadurch gekennzeichnet, dass** das Spreitgewebe (30) eine Dicke von weniger als 150, vorzugsweise weniger als 110 µm aufweist.

## Claims

1. Analytical test tape (20), in particular for use in a test tape cassette (14), having a carrier tape that can be wound onto a spool and a plurality of test elements (24) that are distributed on the carrier tape in the tape longitudinal direction and have a spreading fabric (30) for applying body fluid and an underlying reagent layer (36) for detecting an analyte in the body fluid, wherein the spreading fabric (30) is formed from fabric threads (44, 46) that are crossed in a grid shape, wherein the bending stiffness of the spreading fabric (30) is sectionally modified so that the bending stiffness is high in the tape transverse direction and low in the tape longitudinal direction, wherein the bending stiffness is modified by one of the following possibilities:
- the use of fabric threads (44,46) of different thicknesses in appropriate areas of the fabric;
- the additional coating in certain areas of the fabric threads (44,46);
- the thinning or local weakening of one or more fabric threads (44,46);
- the use of multifilament fabric threads (44,46).

2. Analytical test tape (20) according to claim 1, **characterized in that** the bending stiffness of the spreading fabric (30) is adapted to reduce the gap between the spreading fabric (30) and the reagent layer (36) of a test field (20) provided for the application of body fluid.

3. Analytical test tape (20) according to claim 1 or 2, **characterized in that** the fabric threads (44, 46) in parts have reduced thread cross-sections, preferably by laser ablation or etching or mechanical removal of material.

4. Analytical test tape (20) according to one of claims 1 to 3, **characterized in that** the fabric threads (44, 46) have a non-uniform design due to different thread sizes, coatings or filament structures in order to modify the bending stiffness.

5. Analytical test tape (20) according to one of claims 1 to 4, **characterized in that** the fabric geometry of the spreading fabric (30) is locally varied.

6. Analytical test tape (20) according to one of claims 1 to 5, **characterized in that** the spreading fabric (30) is wider than the reagent layer (36) and is supported flat on the test tape (20) by spacers (32) in the region of its projecting side edges.

7. Analytical test tape (20) according to one of claims 1 to 6, **characterized in that** the spreading fabric (30) has a thickness of less than 150, preferably less than 110 µm.

## Revendications

1. Bande d'essai (20) analytique, destinée, en particulier à être utilisée dans une cassette de bande d'essai (14), comportant une bande porteuse pouvant être enroulée sur une bobine et une pluralité d'éléments d'essai (24) disposés sur la bande porteuse de manière répartie dans le sens longitudinal de bande, lesquels comportent un tissu extensible (30) destiné à l'application du fluide corporel et une couche réactive (36), se trouvant en dessous de celui-ci, permettant de détecter un analyte dans ledit fluide corporel, dans laquelle le tissu extensible (30) est formé à partir de fils du tissu (44, 46) croisés en forme de grille, dans laquelle la résistance à la flexion du tissu extensible (30) est modifiée par zones de telle sorte que ladite résistance à la flexion est élevée dans le sens transversal de bande et faible dans le sens longitudinal de bande ; dans laquelle la résistance à la flexion est modifiée par l'une des possibilités suivantes :
- l'utilisation des fils du tissu (44, 46) de différentes épaisseurs dans les zones correspondantes du tissu ;
- le revêtement supplémentaire dans les zones déterminées des fils du tissu (44, 46) ;
- l'amincissement ou l'affaiblissement local de l'au moins un fil du tissu (44, 46) ;
- l'utilisation de fils du tissu (44, 46) multifilament.

2. Bande d'essai (20) analytique selon la revendication 1, **caractérisée en ce que** la résistance à la flexion du tissu extensible (30) est ajustée pour réduire la distance entre le tissu extensible (30) et la couche réactive (36) d'un champ d'essai (20) fourni pour l'application de fluide corporel.

3. Bande d'essai (20) analytique selon la revendication 1 ou 2, **caractérisée en ce que** les fils du tissu (44, 46) comportent des sections transversales de fil réduites par endroits, de préférence par ablation au laser ou par décapage ou par enlèvement mécanique de matière.

4. Bande d'essai (20) analytique selon l'une quelconque des revendications 1 à 3, **caractérisée en ce que** les fils du tissu (44, 46) sont conçus non uniforme en raison d'épaisseurs de fils, de revêtements ou de structures de filaments différents pour modifier la résistance à la flexion.

5. Bande d'essai (20) analytique selon l'une quelconque des revendications 1 à 4, **caractérisée en ce que** la géométrie du tissu du tissu extensible (30) est variée localement.

6. Bande d'essai (20) analytique selon l'une quelconque des revendications 1 à 5, **caractérisée en ce que** le tissu extensible (30) est plus large que la couche réactive (36) et est soutenu uniformément sur la bande d'essai (20), dans la zone de ses bords latéraux en saillie, par des entretoises (32).

7. Bande d'essai (20) analytique selon l'une quelconque des revendications 1 à 6, **caractérisée en ce que** le tissu extensible (30) présente une épaisseur inférieure à 150, de préférence inférieure à 110 µm.
